# EUROPEAN PATENT APPLICATION

(11) **EP 2 042 600 A1**
(43) Date of publication of application: **01.04.2009**
(21) Application number: 07291166.2
(22) Date of filing: 28.09.2007
(51) Int. Cl.: C12N 15/49, C12N 15/85, C12N 5/10, C07K 14/16, A61K 39/21, G01N 33/569

(54) **A public and immunogenic CD4+ T Cell epitope of the hiv tat protein and its applications**

(71) Applicant: COMMISSARIAT A L'ENERGIE ATOMIQUE, 75015 Paris (FR)
(72) Inventor: Maillere, Bernard, 78000 Versailles (FR); Castelli, Florence, 92120 Montrouge (FR); Houitte, Diane, 78210 Saint Cyr l'Ecole (FR); Munier, Gaetan, 91120 Palaiseau (FR)
(74) Representative: Leblois-Préhaud, Hélène Marthe Georgette

(57) **Abstract**

An isolated, immunogenic Tat peptide as anti-HIV vaccine, said peptide consisting of 11 to 15 consecutive amino acids of Tat 41-55 comprising at least positions 44 to 50, said Tat peptide consisting essentially of a T CD4+ cell epitope and having a binding activity for at least three of the most prevalent HLA-DR molecules in the Caucasian population, and its applications for HIV vaccine development.

SEQUENCE LISTING

<110>  COMMISSARIAT A L' ENERGIE ATOMIQUE

<120>  A PUBLIC AND IMMUNOGENIC CD4+ T CELL EPITOPE OF THE HIV TAT
       PROTEIN AND ITS APPLICATIONS

<130>  263EP214

<160>  17

<170>  PatentIn version 3.3

<210>  1
<211>  86
<212>  PRT
<213>  Human immunodeficiency virus

<400>  1

Met Glu Pro Val Asp Pro Arg Leu Glu Pro Trp Lys His Pro Gly Ser
1               5                   10                  15

Gln Pro Lys Thr Ala Cys Thr Thr Cys Tyr Cys Lys Lys Cys Cys Phe
            20                  25                  30

His Cys Gln Val Cys Phe Thr Thr Lys Ala Leu Gly Ile Ser Tyr Gly
        35                  40                  45

Arg Lys Lys Arg Arg Gln Arg Arg Arg Pro Pro Gln Gly Ser Gln Thr
    50                  55                  60

His Gln Val Ser Leu Ser Lys Gln Pro Thr Ser Gln Pro Arg Gly Asp
65                  70                  75                  80

Pro Thr Gly Pro Lys Glu
                85

<210>  2
<211>  7
<212>  PRT
<213>  artificial sequence

<220>
<223>  Tat 44-50

<400>  2

Gly Ile Ser Tyr Gly Arg Lys
1               5

<210>  3
<211>  15
<212>  PRT
<213>  artificial seequence

<220>
<223>  HIV-1 group M subtype B consensus Tat 41-55

<400>  3

Lys Gly Leu Gly Ile Ser Tyr Gly Arg Lys Lys Arg Arg Gln Arg
1               5                   10                  15


<210>  4
<211>  15
<212>  PRT
<213>  artificial sequence

<220>
<223>  HIV-1 group M subtype B variant A Tat 41-55

<400>  4

Lys Ala Leu Gly Ile Ser Tyr Gly Arg Lys Lys Arg Arg Gln Arg
1               5                   10                  15


<210>  5
<211>  9
<212>  PRT
<213>  artificial sequence

<220>
<223>  Tat 49-57 (PTD)

<400>  5

Arg Lys Lys Arg Arg Gln Arg Arg Arg
1               5


<210>  6
<211>  26
<212>  PRT
<213>  artificial sequence

<220>
<223>  Tat 30-55

<400>  6

Cys Cys Phe His Cys Gln Val Cys Phe Thr Thr Lys Ala Leu Gly Ile
1               5                   10                  15


Ser Tyr Gly Arg Lys Lys Arg Arg Gln Arg
            20                  25


<210>  7
<211>  32
<212>  PRT
<213>  artificial sequence

<220>
<223>  Tat 30-61

<400>  7

Cys Cys Phe His Cys Gln Val Cys Phe Thr Thr Lys Ala Leu Gly Ile
1               5                   10                  15


Ser Tyr Gly Arg Lys Lys Arg Arg Gln Arg Arg Arg Pro Pro Gln Gly
            20                  25                  30

<210>  8
<211>  13
<212>  PRT
<213>  artificial sequence

<220>
<223>  HA 306-318

<400>  8

Pro Lys Tyr Val Lys Gln Asn Thr Leu Lys Leu Ala Thr
1               5                   10


<210>  9
<211>  13
<212>  PRT
<213>  artificial sequence

<220>
<223>  YKL

<400>  9

Ala Ala Tyr Ala Ala Ala Lys Ala Ala Ala Leu Ala Ala
1               5                   10


<210>  10
<211>  15
<212>  PRT
<213>  artificial sequence

<220>
<223>  A3 152-166

<400>  10

Glu Ala Glu Gln Leu Arg Ala Tyr Leu Asp Gly Thr Gly Val Glu
1               5                   10                  15


<210>  11
<211>  15
<212>  PRT
<213>  artificial sequence

<220>
<223>  MT 2-16

<400>  11

Ala Lys Thr Ile Ala Tyr Asp Glu Glu Ala Arg Arg Gly Leu Glu
1               5                   10                  15


<210>  12
<211>  16
<212>  PRT
<213>  artificial sequence

<220>
<223>  B1 21-36

<400>  12

Thr Glu Arg Val Arg Leu Val Thr Arg His Ile Tyr Asn Arg Glu Glu
1               5                   10                  15

<210>  13
<211>  20
<212>  PRT
<213>  artificial sequence

<220>
<223>  LOL 191-210

<400>  13

Glu Ser Trp Gly Ala Val Trp Arg Ile Asp Thr Pro Asp Lys Leu Thr
1               5                   10                  15

Gly Pro Phe Thr
            20

<210>  14
<211>  14
<212>  PRT
<213>  artificial sequence

<220>
<223>  E2/E168

<400>  14

Ala Gly Asp Leu Leu Ala Ile Glu Thr Asp Lys Ala Thr Ile
1               5                   10

<210>  15
<211>  15
<212>  PRT
<213>  artificial sequence

<220>
<223>  HIV- group M subtype B variant C Tat 41-55

<400>  15

Lys Gly Leu Gly Ile Ser Tyr Gly Ser Lys Lys Arg Arg Gln Arg
1               5                   10                  15

<210>  16
<211>  15
<212>  PRT
<213>  artificial sequence

<220>
<223>  HIV-1 group M subtype B variant D Tat 41-55

<400>  16

Lys Ala Leu Gly Ile Ser Asn Gly Arg Lys Lys Arg Arg Gln Arg
1               5                   10                  15

<210>  17
<211>  15
<212>  PRT
<213>  artificial sequence

<220>

<223>  HIV-1 group M subtype B variant E Tat 41-55

<400>  17

Lys Gly Leu Gly Ile Tyr Tyr Gly Arg Lys Lys Arg Arg Gln Arg
1               5                   10                  15

## Description

The invention relates to a public and immunogenic CD4+ T cell epitope of the HIV Tat protein and its applications, in particular for HIV vaccine development.

Twenty years after the discovery of HIV, an effective prophylactic or therapeutic vaccine is still not available. As illustrated by the recent unsuccessful phase III study, induction of neutralizing antibodies specific to the Env protein appears difficult and is limited by the large diversity of HIV isolates (Flynn et al., J. Infect. Dis., 2005, 191:654-665). Cellular responses induced by structural antigens (Env, Gag, Pol) are partially protective in preclinical studies but do not give rise to sterilizing immunity (Letvin et al., Annu. Rev. Immunol., 2002, 20:73-99). Actual approaches are mainly based on the introduction of structural antigens into viral vectors, their combination with strong adjuvants or their injection as DNA vaccine (Johnston, M. I. and A. S. Fauci, N. Engl. J. Med., 2007, 356:2073-2081). Alternative or complementary approaches aim at neutralizing accessory or regulatory proteins because they disturb the immune system and counteract its beneficial role (Ferrantelli et al., Curr. Opin. Biotechnol., 2004, 15:543-556; Mascarell et al., J. Virol., 2005, 79:9872-9884; Goepfert et al., Vaccine, 2007, 25:510-518).

Tat is a small regulatory protein that is expressed early in the viral cycle and is essential for viral replication (Fisher et al., Nature, 1986, 320:367-371). The *Tat* gene comprises 2 exons encoding a protein of 99 to 103 amino acids depending on the HIV strains. Exon 1, which encodes the first 72 amino acids, has a complete transactivation activity and comprises 5 domains: (1) the N-terminal domain (positions 1 to 21), which is important for the interaction with cell proteins, (2) the cysteine-rich domain (positions 22 to 37) containing 7 cysteine residues among which 6 are strongly conserved, which domain is involved in transactivation, (3) the central (core) domain corresponding to positions 38 to 48, also involved in transactivation, (4) the basic domain (positions 49 to 57), which comprises the sequences involved in nuclear localization, transcellular transport (protein transduction domain (PTD) or cell penetration domain) and binding to the TAR (*Trans-activation response*) element of the viral LTR (*Long Terminal Repeat*), and which is also involved in the binding of Tat to heparin, and (5) the glutamine-rich domain (positions 50 to 72). Exon 2, which is variable in size, encodes the C-terminal domain (positions 73 to the C-terminal end) which does not have transactivation activity but contains the RGD motif (arginine-glycine-aspartate; positions 78 to 80), required for Tat binding to integrin receptors. In addition to the Tat protein of 99 to 103 amino acids, there also exists a truncated Tat protein of 86 amino acids, produced *in vitro* by generation of a stop codon at position 87, due to the mutation of HIV during passage in cell culture. Besides its transactivating activity, Tat exerts a wide range of activities. It diminishes T cell proliferation (Viscidi et al., Science, 1989, 246:1606-1608) modulates generation of CTL epitopes and provokes immunosuppression (Cohen et al., Proc. Natl. Acad. Sci. USA., 1999, 96:10842-10847). It also up-regulates expression of chemokine receptors (Secchiero et al., J. Immunol., 1999, 162:2427-2431), enhances dendritic cell maturation (Fanales-Belasio et al., J. Immunol., 2002, 168:197-206) and exhibits an adjuvant activity (Kittiworakarn et al., J. Biol. Chem., 2006, 281:3105-3115). The Tat protein could be targeted by CD8+ T cells in seropositive donors (Novitsky et al., J. Virol., 2001, 75:9210-9228; Addo et al., Proc Natl Acad Sci U S A, 2001, 98:1781-1786; Novitsky et al., J. Virol., 2002, 76:10155-10168; Cao et al., J. Immunol., 2003, 171:3837-3846; Cao et al., J. Virol., 2003, 77:6867-6878) and hence constitutes potential candidate to elicit HIV specific cellular response. Multiple forms of Tat including peptides elicit neutralizing antibodies in monkeys and lead to promising but controversial results in term of protection (Cafaro et al., Nat. Med; 1999, 5:643-650; Allen et al., J. Virol., 2002, 76:4108-4112; Silvera et al., J. Virol., 2002 76:3800-3809 ; Belliard et al., Vaccine, 2005, 23:1399-1407; Blazevic et al., J. Acquir. Immune Defic. Syndr., 1993, 6:881-890).

The CD4⁺ T cells possess a rearranged T receptor which allows them to selectively recognize peptide fragments derived from the degradation of the antigen by the presenting cells and presented by molecules of the Major Histocompatibility Complex class II (MHC II) or Human Leukocyte antigen class II (HLA II) in humans (Germain et al., Annu. Rev. Immunol., 1993, 11, 403-). The determinants carried by these peptide fragments and recognized by the T cells are called T-cell epitopes. T CD4⁺ epitopes are small peptides possessing a sequence of variable length which is able to bind specifically to HLA II molecules; although peptides naturally bound to HLA II molecules are 13 to 25 amino acids long, smaller peptides (11 to 20 amino acids) still bind to HLA II molecules *in vitro* and *in vivo.* It is well-known that like the native antigen, a peptide comprising a T CD4+ epitope is able to stimulate specific CD4+ cells *in vitro* or to induce such cells *in vivo.* Such peptide is therefore sufficient to induce a CD4+ T cell response.

Therefore, the immunological data regarding Tat and the requirement of CD4+ T cell to sustain both humoral and cellular response, make it possible to consider such immunogenic peptides specific for Tat and capable of inducing a strong CD4+ T cell response for the preparation of an anti-HIV vaccine in human, especially in association with B or T epitopes specific for HIV antigens.

In addition, such peptides which recognize HIV specific CD4+ T cells are also useful as reagents for the detection of the immune state of an individual with respect to HIV virus infection, HIV antiviral therapy or HIV vaccination. HIV specific T CD4+ epitopes are usually identified by approaches based on detection of HIV specific T cells in asymptomatic or HAART treated seropositive patients (Wilson et al., J. Virol., 2001, 75:4195-4207; Malhotra et al., J. Clin. Invest., 2001, 107:505-517; Kaufmann et al., J. Virol., 2004, 78:4463-4477; Malhotra et al., J. Virol., 2003, 77:2663-2674; Younes et al., J. Exp. Med., 2003). Few studies exist on the CD4+ T cell epitopes specific to the Tat protein in human and they have been performed using cells collected in seropositive donors (Goepfert et al., Vaccine, 2007, 25:510-518; Blazevic et al., J. Acquir. Immune Defic. Syndr., 1993, 6:881-890; Betts et al., J. Virol., 2001, 75:11983-11991; Ranki et al., J. Acquir. Immune Defic. Syndr., 1997, 11:132-133). Three peptides (Tat 17-32, Tat 33-48 and Tat 65-80) were significantly recognized by the CD4+ T cells from HIV-infected patients (Blazevic et al., J. Acquir. Immune Defic. Syndr., 1993, 6:881-890; Ranki et al., J. Acquir. Immune Defic. Syndr., 1997, 11:132-133).

However, the data provided by these studies contribute to the understanding of HIV immunopathology and to the research of correlates of protection. However, CD4+ T cell response in HIV patients could be weak (Pitcher et al., Nat Med, 1999, 5:518-525) and could focus on a limited number of T cell epitopes (Malhotra et al., J. Virol., 2003, 77:2663-2674). HIV virus load appeared to alter proliferative CD4+ T cell response (Younes et al., J. Exp. Med., 2003; Palmer et al., J. Immunol., 2004, 172:3337-3347). As recently shown for the vaccinia virus, virus infection recruited a limited numbers of immunodominant T cell epitopes and impaired the selection of subdominant ones, although the latter, by definition, induced a virus-specific response (Assarsson et al., J. Immunol., 2007, 178:7890-7901). As a result, relevant epitopes are missed by these approaches and potential of virus components for vaccine design are underestimated.

The peptides which are effectively recognized by T CD4⁺ lymphocytes are difficult to identify due to the high degree of polymorphism of HLA II molecules. The MHC class II molecules are αβ heterodimers (alpha and beta chains) capable of binding a large repertoire of peptides having very different sequences, which allow them to present several peptides per antigen to the T cells. There are four different types of MHC II molecules per individual (2 HLA-DR, 1 HLA-DQ and 1 HLA-DP), named after the allele coding for the beta chain which is the most polymorphic. The HLA-DR molecule is very polymorphic. Three alleles have been recorded for the alpha chain whereas at least 458 alleles have been recorded for the beta chain encoded by the DRB1 gene (HLA-DR1), which is the most highly expressed. The molecules encoded by the DRB3, DRB4 and DRB5 genes (HLA-DRB3, HLA-DRB4 and HLA-DRB5) are less polymorphic. The HLA isoforms (or antigens or types) encoded by these alleles possess binding properties that are different from one another, which implies that they can bind different peptides of the same antigen. The peptide-binding mode of HLA II molecules is more complex than that of HLA I molecules and there is currently no reliable computer program for predicting the sequence of an HLA II ligand (Reche, P.A. and E.L. Reinherz, Nucleic Acids Res., 2005, 33, w138-w142). Although HLA I and HLA II molecules have a similar tridimentional structure, the binding site of HLA II molecules is a groove that is opened at both extremities. For these reasons, HLA II restricted peptides have variable length, usually comprised between 13 and 25 amino acids. This groove is characterized by five pockets of specificity (P1, P4, P6, P7 and P9, corresponding to the positions of the peptide residues which they accommodate) which comprise polymorphic residues. Since the differences between the HLA II molecules are mainly localized in the pockets of the HLA II molecule binding site, they determine the repertoire of peptides which bind to each HLA II molecule.

Due to the high degree of polymorphism of the HLA molecules, the set of peptides from an antigen, which are recognized by each individual depends on the HLA molecules or HLA type which characterize said individual. Since there is a large number of HLA II isoforms, in a given antigen, there is a large repertoire of peptides able to stimulate a specific T CD4+ response, each one being specific for a different HLA isoform. Therefore, a peptide able to stimulate a T CD4+ response in one individual may be inactive in the majority of other individuals because the latter do not recognize the antigen with the same epitope. Hence, because of its small size, CD4+ T cell response specific for Tat protein is expected to largely depend on the control by HLA molecules. As illustrated by pioneering studies on the genetic control of immune response in mice (Schwartz, R. H., and W. E. Paul, J. Exp. Med., 1976, 143:529-540; Okuda et al., J. Immunol., 1978, 121:866-868) and humans (Mann et al., J. Clin. Invest., 1983, 72:1130-1138; O'Brien et al., Immunology, 1995, 86:176-182), small size proteins lead to high and low responders because of the presence or absence of peptide sequences able to be presented to T cells by the MHC II molecules (Buus et al., Science, 1987, 235:1353-1358; Guillet et al., Science, 1987, 235:865-870).

Despite of a high number of HLA molecules whose repartition is not homogeneous worldwide, some alleles are predominant in a given population. In addition the binding motifs of some HLA-DR molecule are identical and a peptide can therefore bind several HLA-DR molecules. Furthermore, some T CD4+ epitopes overlap each other so that a peptide sequence may comprise several T CD4+ epitopes.

For example, ten HLA-DR molecules are preponderant in Europe and in the United States and cover the vast majority of the population: DR1, DR3, DR4, DR7, DR11, DR13, DR15 and DRB3, DRB4 and DRB5 (Table I).

**Tableau I : HLA-DR molecules allelic and phenotypic frequencies**

| **Alleles** | **Europe** | | **USA** | | **Africa** | **Asia** | |
|---|---|---|---|---|---|---|---|
| | **France** | **Germany** | **Caucasian** | **Afro-american** | **Senegal** | **India** | **Japan** |
| **DRB1*0101** | **9,3**/17,7 | **6,7**/13 | **7,3**/14,1 | 1,9/3,8 | 0,6/1,2 | 3,8/7,5 | 4,9/9,6 |
| **DRB1*0401** | **5,6**/10,9 | **8,1**/15,5 | **6,7**/13 | 1,5/3,0 | 0/0 | 0,9/1,8 | 0/0 |
| **DRB1*1101** | **9,2**/17,6 | **9,2**/17,6 | **4,4**/8,6 | **8,2**/15,7 | **9,3/17,7** | 0,9/1,8 | 2/4 |
| **DRB1*0701** | **14,0**/26 | **12,3**/23,1 | **14,4**/26,7 | **9,8**/18,6 | **7,8/15** | **13/24,3** | 0,6/1,2 |
| **DRB1*0301** | **10,9**/20,6 | **9,4**/17,9 | **9,5**/18,1 | **7**/13,5 | **10,2/19,4** | **5,3/10,3** | 0,4/0,8 |
| **DRB1*1301** | **6,0**/11,6 | 4,5/8,8 | 5,1/9,9 | 4,2/8,2 | 4,7/9,2 | **6,3/12,2** | 0,7/1,4 |
| **DRB1*1501** | **8,0/14,4** | **7,8/15** | 10,3/19,5 | **8,6**/16,5 | 0/0 | **12,1/22,7** | **9,1/17,4** |
| **TOTAL** | **63,0/86,3** | 58,0/82,**4** | 57,7/82,1 | 41,2/65,**4** | 32,/54,66 | 42,3/66,7 | 17,7/32,3 |
| | | | | | | | |
| **DRB5*0101** | **7,9/15,2** | 4,6/9 | 2,4/4,7 | **10,4**/19,7 | 0/0 | 0/0 | **5,6**/10,9 |
| **DRB3*0101** | **9,2**/17,6 | **9,8**/18,6 | **10,4**/19,7 | **15,1**/27,9 | **6,9**/13,3 | 4,9/9,6 | **6,5**/12,6 |
| **DRB4*0101** | **28,0/48,2** | **21,1**/37,7 | **19,8**/35,7 | **16,5**/30,3 | **6,9**/13,3 | **24,8**/43,4 | **28,9**/49,4 |
| **TOTAL** | 45,1/69,9 | 35,5/58,4 | 32,6/54,6 | 42,0/66,4 | 13,8/25,7 | 29,7/50,6 | 41,0/65,2 |

| | | | | | | | |
|---|---|---|---|---|---|---|---|
| * the preponderant HLA-DR molecules (allelic frequence > 5 %) are in bold. ** nd: not determined. | | | | | | | |

For example, in France, which is representative of the Caucasian population, seven DRB1 alleles only, exceed 5 % (DRB1*0101, DRB1^{*}0301, DRB1^{*}0401, DRB1^{*}0701, DRB1^{*}1101, DRB1^{*}1301 and DRB*1501). They represent, on their own, 63 % of the French population. These same alleles are also the most abundant in the other Caucasian populations. Their frequencies vary from 53 % (in Spain) to 82 % (in Denmark). For the United States and Canada, they represent 58 and 55 % of the population, respectively. They therefore represent, on their own, 53 to 82 % of the alleles in Caucasian populations. In addition, some of the most frequent DRB1 alleles in the Caucasian population are also frequent in Africa (DRB1*0310, *0701 and *1101), India (DRB1*0301,*0701, *1301 and *1501) and Japan (DRB1*1501).

The HLA-DRB3, -DRB4 and -DRB5 molecules which are less polymorphic than those encoded by HLA-DRB1 are also present at high allelic frequencies in Europe, USA and Japon (Table I). For example, the allelic frequency in France is 9.2 % for DRB3*0101, 28.4 % for DRB4*0101 and 7.9 % for DRB5*0101. They cover, on their own, 45 % of the allelic frequency in France.

Therefore the peptides present in the sequence of an antigen and which bind most of these HLA-DR alleles therefore include the T epitopes of the majority of the population.

To shed some light on the CD4+ T cell stimulating properties of Tat protein, the inventors have characterized its HLA-DR restricted CD4+ T cell epitopes and identified HLA-DR alleles that contribute to the T cell response specific for Tat in human.

The experimental approach was based on the combination of binding assays to preponderant HLA-DR molecules and of primary stimulation assays using CD4+ T lymphocytes collected in seronegative donors. The use of binding assays to select promiscuous binders is consistent with many studies supporting the role of good affinity in the T cell stimulating properties of peptides (Wilson et al., J. Virol., 2001, 75:4195-4207; Zarour et al., Cancer Res, 2002, 62:213-218; Janjic et al., J. Immunol., 2006, 177:2717-2727). It is more robust than binding prediction and it is especially adapted to relatively short protein sequences. *In vitro* priming experiments appear as a suitable way to evaluate peptide immunogenicity because it tentatively reproduces in *vitro* the T cell repertoire conditions encountered *in vivo* upon injection of prophylactic vaccines. It has been successfully used to identify T cell epitopes from tumor (Chaux et al., J Exp Med, 1999, 189:767-778; Zarour et al., Proc Natl Acad Sci U S A, 2000, 97:400-405) and HIV antigens (Venturini et al., J. Virol., 2002,76:6987-6999; Pancre et al., Clin Exp Immunol, 2002, 129:429-437) and to assess immunogenicity of therapeutic proteins (Tangri et al., J. Immunol., 2005, 174:3187-3196). Identified T cell stimulating peptides by this approach have been found to be immunogenic in vaccination trials of naive individuals (Jenkins et al., J. Immunol., 1999, 162:560-567; Schuler-Thurner et al., J. Exp. Med., 2002, 195:1279-1288), contrary to approaches based on detection of HIV specific T cells in asymptomatic or HAART treated seropositive patients since relevant epitopes are missed by these approaches and potential of virus components for vaccine design are underestimated.

Therefore, peptides covering the Tat protein sequence were investigated for their capacity to bind to the 10 most prevalent HLA-DR molecules in the Caucasian population which are also prevalent worldwide. Four peptides (Tat 24-38, Tat 30-44, Tat 34-48 and Tat 41-55) were selected based on their binding activity to preponderant HLA-DR molecules. Primary stimulation assays using CD4+ T lymphocytes collected in seronegative donors showed that the peptide specific T cell response was mainly focused to one CD4+ T cell epitope only. The Tat 41-55 induced multiple CD4+ T cell lines in HLA unrelated donors and is presented to T lymphocytes by multiple HLA-DR molecules in agreement with its binding capacity. Tat 41-55 peptide-specific T cell lines recognized the native antigen processed by the dendritic cells demonstrating that the Tat 41-55 peptide represent a peptide naturally processed by the dendritic cells. Based on the T cell lines specificity data, it is expected that at least HLA-DR11, DR3, DR13 and DR15/DRB5 individuals, which represent approximately 57 % of the Caucasian population, are responders to Tat 41-55 immunization. Furthermore, Tat 41-55 exhibits cross-reactivity with natural variants of HIV-1 indicating that it is able to induce a T CD4+ response towards HIV-1 natural variants.

By contrast, Tat 65-79 which is close to the previously described Tat 65-80 peptide was a low binder to all ten most prevalent HLA-DR molecules worldwide. Furthermore, Tat 24-38, Tat 30-44 and Tat 34-48 (all included in the Tat 17-48 sequence spanning the previously described Tat 17-32 and Tat 33-48 peptides) appeared weakly immunogenic compared to 41-55, although they were high binders to at least three of the ten most prevalent HLA-DR molecules worldwide. In addition, by contrast to Tat 41-55, the T cell lines specific to these peptides did not recognize the native antigen processed by the dendritic cells demonstrating that Tat 24-38, Tat 30-44 and Tat 34-48 did not seem to correspond to naturally processed peptides.

The invention relates to an isolated, immunogenic Tat peptide as anti-HIV vaccine, wherein said peptide consists of 11 to 15 consecutive amino acids of the peptidic fragment from positions 41 to 55 of HIV Tat protein (Tat 41-55) comprising at least positions 44 to 50, and wherein said Tat peptide consists essentially of a T CD4+ cell epitope and has a binding activity for at least three of the most prevalent HLA-DR molecules in the Caucasian population.

In the present invention, "HLA-DR molecules" means HLA molecules which are encoded by different HLA-DRB genes or different alleles from these genes.

In the present invention "prevalent", "predominant", "preponderant" HLA-DR molecule refers to an HLA-DR molecule whose allelic frequency exceeds 5 % in the Caucasian population, as indicated in Table I.

In the present invention, a "public epitope" refers to an epitope which is recognized by the majority of individuals.

The Tat peptide according to the invention has the following properties:

### - HLA-DR binding activity to prevalent HLA-DR molecules

The Tat peptide is a high binder to HLA-DR molecules. The HLA-DR binding activity of the peptide is assessed in a competition binding assay with a labelled peptide which serves as tracer, following the procedures described in the US Patent 6,649,166 and illustrated for example in the International PCT Applications WO 2004/014936, WO 2007/036638, WO 2006/082313 and WO2006/075253. The activity of each peptide is characterized by the concentration of peptide (nM) which inhibits the binding of the biotinylated peptide tracer by 50 % (IC₅₀). It may also be expressed as relative activity: ratio of the IC₅₀ of the peptide by the IC₅₀ of the reference peptide (unlabeled form of the tracer, which is a high binder to the HLA-DR molecule). For example a binding affinity of less than 1000 nM or a relative binding activity of less than 100 corresponds to a high binder to the HLA-DR molecule.

The Tat peptide binds to at least three of the most prevalent HLA-DR molecules in the Caucasian population, as indicated in Table I. However, some of the HLA-DR molecules which are preponderant in the Caucasian population are also preponderant in other populations (Table I). Therefore, the peptide of the invention is not limited to a use in Europe or North America but can be used worldwide. The Tat peptide may be presented by HLA-DR molecules which are preponderant in the worldwide population since it is presented by DR11 which is preponderant in Europe and Africa, DR3 which is preponderant in Europe, North America, Africa and India, DR13 which is preponderant in Europe, North America and India, DR15 which is preponderant in Europe, North America, and Asia and DRB5 which is preponderant in Europe, North America (Afro-American) and Japan (Table I).

### - immunogenicity

The Tat peptide of the invention is recognized by T CD4+ lymphocytes in the majority of individuals (public T CD4+ epitope) since it is presented by HLA-DR molecules which are the most prevalent in the Caucasian population and also prevalent in the other populations worldwide. The Tat peptide is able to induce efficiently HIV specific CD4+ T lymphocytes from precursors which are present in the majority of naive (HIV seronegative) or infected (HIV seropositive) subjects. It is also able to stimulate such HIV specific T lymphocytes in the majority of infected subjects. Furthermore, the CD4+ T lymphocytes which are induced by the Tat peptide recognize the native antigen, naturally processed by the dendritic cells as well as natural variants of said peptide.

Therefore, such peptide represent a potential candidate for the prophylactic or therapeutic vaccination against HIV infection which is particularly interesting since it is able to induce an HIV specific T CD4+ response in the majority of individuals in the population.

In addition, the peptide sequence which is relatively conserved in the natural variants of HIV-1 and exhibits cross-reactivity with T CD4+ epitopes recognized by natural variants of HIV is also useful as reagent for the detection of the immune state of an individual with respect to HIV virus infection, HIV antiviral therapy or HIV vaccination.

The immunogenicity of the peptide may be assayed from peripheral blood mononuclear cells (PBMC), by any technique well-known in the art, such as for example: lymphocyte proliferation assay or a cytokine assay (IFN-γ, IL-2, IL-4, IL-10).

The Tat peptide according to the invention comprises a T CD4+ epitope consisting of a 11 to 15 amino acid sequence (11, 12, 13, 14 or 15 amino acids) comprising a central region of 9 amino acids including the P1, P4, P6, P7, P9 anchor residues for HLA-DR binding, flanked by two to four amino acids at the amino-terminal extremity, and eventually also at the carboxy-terminal extremity. The P1 anchor residue for HLA-DR may be in position 43 or 45 of Tat amino acid sequence. For example, it is L43 or I45.

The invention encompasses Tat peptides derived from any HIV (HIV-1 or HIV-2) isolate, including the natural variants of HIV, isolated from HIV infected subjects. The HIV-1 isolates are classified in three groups (M, N(non-M, non-O) and O) which are divided in subtypes or clades based on their genetic similarity (Env gene); within group M there are known to be at least nine genetically distinct subtypes (or clades) of HIV-1: A, B, C, D, F, G, H, J and K. The sequences of HIV isolates are available from the Los Alabamos National Laboratory database (LANL; http://www.hiv.lanl.gov). Consensus sequences derived from the HIV-1 variant sequences are also available, in particular for the M group and some of its subtypes (http://hiv.lanl.gov/content/hiv-db/CONSENSUS/M GROUP/Consensus.html).

In the present invention, the positions of the Tat peptide are indicated by reference to Tat protein sequence SEQ ID NO: 1 which is that of the LAI/IIIB isolate, corresponding to variant A of HIV-1 M group subtype B. From these indications, one skilled in the art will find easily the corresponding positions in other HIV isolates which might vary of few amino acids because of small insertions/deletions in Tat protein amino acid sequence.

The invention encompasses also the modified Tat peptides derived from the Tat peptides as defined above, by introduction of any modification in the amino acid residues, the peptide bond or the peptide extremities, providing that the modified peptide is still active for HLA-DR binding and T CD4⁺ recognition. The modifications which are introduced by procedures well-known in the art include with no limitation: mutation (insertion, deletion, substitution) of one or more amino acids in the peptide sequence; addition of an amino acid fusion moiety; substitution of amino acid residues by non-natural amino acids (D-amino-acids or non-amino acid analogs); modification of the peptide bond (retro or retro-inverso type bond; methyleneamino, carba, ketomethylene or methyleneoxy type bond); cyclization; addition of chemical groups to the side chains (lipids, oligo-or -polysaccharides); coupling to an appropriate carrier; fusion to a peptide sequence of interest (epitope of interest for the vaccination; tag useful for the purification of the peptide, in particular a protease-cleavable tag); fusion to any protein of interest, for example to the alpha or beta chain of an HLA -DR molecule or to the extracellular domain of said chain; coupling to a molecule of interest including an antigen or a label, for example a fluorochrome. These modifications are for increasing the stability, the solubility, the immunogenicity or for facilitating the purification or the detection of the peptide or of the CD4+ T cells that are specific to the peptide.

In particular, the invention encompasses the synthetic variants obtained by mutation (insertion, deletion, substitution) of one or more amino acids in the Tat peptide sequence providing that the modified peptide is still active for HLA-DR binding and T CD4⁺ recognition. Such variant peptides are prepared according to procedures well-known in the art. For example, the mode of binding of peptides to HLA-DR molecules can be determined according to the protocols described in the US Patent 6,649,166. The determination of the peptide residues which are accommodated by the pockets P1, P4, P6, P7, P9 of HLA-DR molecules (P1, P4, P6, P7 and P9 residues or anchor residues) makes it possible to introduce mutations at these positions to modify (increase or decrease) the binding affinity of said peptide to HLA molecules, and eventually modify the specificity of the peptide towards HLA-DR. Examples of said mutations and their effect on the HLA-DR binding activity of the peptides are presented in the US Patent 6,649,166. HLA-DR binding motifs are also described in Sturnolo et al., Nat. Biotech., 1999, 17, 533-534 and Rammensee et al., Immunogenetics, 1995, 41, 178-228. Other residue(s), different from the anchor residues, may also be mutated to modulate T CD4+ cell recognition, for example to increase cross-reactivity with natural variants of the Tat peptide

According to a preferred embodiment of said Tat peptide, the amino acid sequence from positions 44 to 50 is GISYGRK (SEQ ID NO: 2).

According to another preferred embodiment of said Tat peptide, it is selected from the group consisting of: Tat 41-51, Tat 41-52, Tat 41-53, Tat 41-54, Tat 41-55, Tat 43-53, Tat 43-54 and Tat 43-55.

According to another preferred embodiment of said Tat peptide, its sequence is from HIV-1 group M subtype B consensus sequence. Preferably, it is of the sequence SEQ ID NO: 3.

According to another preferred embodiment of said Tat peptide, its sequence is from HIV-1 group M subtype B variant A sequence. Preferably, it is of the sequence SEQ ID NO: 4.

According to another preferred embodiment of said Tat peptide, it binds to at least three HLA-DR molecules selected from the group consisting of: HLA-DR1, HLA-DR3, HLA-DR4, HLA-DR7, HLA-DR11, HLA-DR13, HLA-DR15, HLA-DRB3, HLA-DRB4 and HLA-DRB5.

More preferably, the HLA-DR molecules are selected from the group consisting of: HLA-DR3, HLA-DR11, HLA-DR13 and HLA-DRB5.

According to another more preferred embodiment of said Tat peptide, said HLA-DR molecules are encoded by the HLA alleles: DRB1*0101, DRB1*0301, DRB1*0401, DRB1*0701, DRB1*1101, DRB1*1301, DRB1*1501, DRB3*0101, DRB4*0101 and DRB5*0101, respectively; the binding profile of the peptide of the invention to said ten HLA-DR molecules which are encoded by the most frequent alleles in the Caucasian population, is illustrated in Table II.

The invention provides also a vaccine composition for the prevention or the treatment of HIV infection/AIDS in an individual, comprising at least one immunogenic Tat peptide as defined above or a polynucleotide encoding said peptide, and one pharmaceutically acceptable vehicle, and optionally, at least one pharmaceutically acceptable carrier and/or adjuvant.

The composition comprises advantageously several different peptides, in particular the sequences of other CD4+ or CD8+ T epitopes, or B epitopes, in particular of HIV proteins. It is to note that the Tat T CD4+ epitope according to the present invention includes the B epitope having the sequence Tat 43-50.

The peptides of the composition may be in the form of either a mixture of isolated peptides or a multi-epitopic peptide comprising the sequences of said peptides directly linked to one another via a peptide bond or else separated by exogenous sequences, i.e. sequences other than those present at this position in the sequence of the HIV Tat. Therefore, the multi-epitopic peptide according to the present invention is a synthetic peptide which is different from Tat. Preferably said multi-epitopic peptide is of about 22 to 1000 amino acids, preferably of 20 to 100 amino acids.

The composition may comprise the sequence of one or more epitopes selected from the group consisting of:
- a T CD8+ epitope of an HIV protein (recognized specifically by HIV specific cytotoxic T lymphocytes and presented by HLA I molecules); HIV specific CTL epitopes are well-known in the art and available from the Los Alamos National laboratory data base (LANL; http://www.hiv.lanl.gov/content/immunology/maps/ctl/ctl.pdf).
- a T CD4+ epitope of the HIV Nef protein, in particular an epitope which binds to at least one HLA-DR molecule that is preponderant in the Caucasian population, as described in the International PCT Application WO 02/062834 (Nef 1-36, 66-94, 74-88, 77-91, 137-168, 139-153, 175-190, 182-198, 178-192, 181-195, 183-197, 187-201 and 189-203),
- a T CD4+ epitope of the HIV Env, RT, Vpr or Vif proteins, in particular an epitope which binds to at least one HLA-DP4 molecule, as described in the International PCT Application WO 2006/027468 (RT 338-352, 263-277, 346-360, 403-417, 408-422; Env 31-45, 388-402, 483-497, 620-634, 670-684, 677-691, 684-698, 749-763 and 760-774, Vpr 15-29, 61-75 and Vif 61-75),
- a universal T CD4+ epitope, natural or synthetic, such as Tetanic Toxoid TT 830-846 (O'Sullivan et al., J. Immunol., 1991, 147, 2663-2669), Influenza virus hemagglutinin peptide HA 307-319 (O'Sullivan *et al.,* precited), PADRE peptide (KXVAAWTLKAA; Alexander et al., Immunity, 1994, 1, 751-761), HIV-1 Nef 45-69 and peptides derived from *Plasmodium falciparum* antigens like for example, CS.T3 (Sinigaglia et al., Nature, 1988, 336 :778-780), CSP, SSP2, LSA-1 and EXP-1 (Doolan et al., J. Immunol., 2000, 165 : 1123-1137),
- a B epitope consisting of a saccharide moiety sucre (Alexander *et al.,* precited), said epitope being preferably in the form of a glycopeptide, and
- a B epitope, more particularly a B epitope derived from an HIV protein that is specifically recognized by antibodies directed to HIV; HIV specific B epitopes are well-known in the art and available from the Los Alamos National laboratory data base (http://www.hiv.lanl.gov/content/immunology/maps/ab/ab.pdf).

The combination of the Tat T CD4+ cell epitope with at least one of the above mentioned epitopes makes it possible to induce or to modulate an anti-HIV immune response in the majority of individuals even if their HLA genotype is unknown.

According to another preferred embodiment of said composition, it comprises at least one immunogenic Tat peptide as defined above and at least one other HIV peptide including a T CD8+ and/or a B epitope as defined above, in the form of a peptide mixture, a multi-epitopic peptide or an expression vector encoding said peptides/multi-epitopic peptide.

According to a more preferred embodiment of said composition, it comprises a multi-epitopic peptide including the sequence of the Tat T CD4+ epitope as defined above and at least one overlapping T CD8+ epitope selected from Tat 30-37, Tat 38-47 or Tat 36-50 and Tat 39-55 and/or the overlapping B epitope Tat 44-61.

According to another more preferred embodiment of said composition, it further comprises at least one HIV Nef, Env, RT, Vpr or Vif T CD4+ cell epitope, one universal T CD4+ epitope, and/or one B epitope consisting of a saccharide moiety, as defined above.

According to another preferred embodiment of said composition, it comprises a multi-epitopic peptide which further includes the Tat protein transduction domain (PTD) having the sequence of Tat 49-57, for intracellular addressing of the peptide. An example of Tat PTD sequence is RKKRRQRRR (SEQ ID NO: 5).

An example of preferred composition comprises a multi-epitopic peptide having the sequence of Tat 30-55 or Tat 30-61, for example SEQ ID NO: 6 or 7, respectively. The long fragments 30-55 or 30-61 could elicit both CD4+ and CD8+ responses and hence constitute a potential candidate for epitope-based vaccine. As they are also including at least one B cell epitope, they are also expected to elicit a Tat specific humoral response in human.

The composition of the present invention may comprise a peptide which is in the form of modified peptide or else peptide associated with liposomes or lipids, in particular in the form of lipopeptide.

The lipid component of the lipopeptide is in particular obtained by addition of a lipid unit on an α-amino function of said peptides or on a reactive function of the side chain of an amino acid of the peptide component; it may comprise one or more C₄₋₂₀ fatty acid-derived chains, optionally branched or unsaturated (palmitic acid, oleic acid, linoleic acid, linolenic acid, 2-aminohexadecanoic acid, pimelautide, trimexautide) or a derivative of a steroid. The preferred lipid component is in particular represented by an N^{α}-acetyl-lysine N^{ε}(palmitoyl) group, also called Ac-K(Pam).

The composition of the present invention may comprise a polynucleotide encoding the peptide or multi-epitopic epitope, as defined above. Preferably, said polynucleotide is included in a vector.

A vector refers to a nucleic acid molecule capable of transporting another nucleic acid to which it has been linked. A vector which can be used in the present invention includes, but is not limited to, a viral vector, a plasmid, a RNA vector or a linear or circular DNA or RNA molecule which may consists of a chromosomal, non chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adeno-associated viruses or AAVs), coronavirus, negative strand RNA viruses such as ortho-myxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).

Preferably said vectors are expression vectors, wherein the sequence(s) encoding the immunogenic Tat peptide/multi-epitopic peptide/fusion protein of the invention is placed under control of appropriate transcriptional and translational control elements to permit production or synthesis of said peptide/multi-epitopic peptide/fusion protein. Therefore, said polynucleotide is comprised in an expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Suitable promoters include tissue specific and/or inducible promoters. Examples of inducible promoters are: eukaryotic metallothionine promoter which is induced by increased levels of heavy metals, heat shock promoter which is induced by increased temperature. Examples of tissue specific promoters are skeletal muscle creatine kinase, prostate-specific antigen (PSA), α-antitrypsin protease, human surfactant (SP) A and B proteins, β-casein and acidic whey protein genes.

The choice of the vector depends on their use and on the host cell; viral vectors and "naked"nucleic acid vectors are preferred vectors for expression in mammal cells (human and animal). Use may be made, inter alia, of viral vectors such as adenoviruses, retroviruses, lentiviruses and AAVs, into which the sequence of interest has been inserted beforehand; it is also possible to associate said sequence (isolated or inserted into a plasmid vector) with a substance capable of providing protection for said sequences in the organism or allowing it to cross the host-cell membrane, for example a preparation of liposomes, of lipids or of cationic polymers, or alternatively to inject it directly into the host cell, in the form of naked DNA. For example, the use of naked DNA for immunization constitutes an effective vaccine approach: it consists in injecting into the host organism to be immunized a naked DNA encoding a protein antigen; this DNA allows prolonged synthesis of the antigen by the host cells and also long-lasting presentation of this antigen to the immune system.

The pharmaceutical vehicles are those appropriate to a planned route of administration, which are well known in the art, such as for example saline, sterile water, Ringer's solution, and isotonic sodium chloride solution.

The carrier may advantageously be selected from the group consisting of: uni- or multi-lamellar liposomes, ISCOMS, virosomes, viral pseudo-particules, saponin micelles, saccharid (poly(lactide-co-glycolide)) or gold microspheres and nanoparticules.

The adjuvant may advantageously be selected from the group consisting of: oil emulsion, mineral substances, bacterial extracts, CpG-containing oligonucleotide, saponin, alumina hydroxide, monophosphoryl-lipid A and squalene.

The composition according to the present invention comprises an amount of peptide, multi-epitopic peptide or expression vector necessary to achieve a prophylactic/therapeutic effect which is determined and adjusted depending on factors such as the age, the sex and the weight of the subject.

The peptide, lipopeptide, multi-epitopic peptide or expression vector is generally administered according to known procedures used for vaccination, at dosages and for periods of time effective to induce an anti-HIV immune response. The administration may be by injection (intradermal subcutaneous, intramuscular, intraperitoneal or intravenous), by oral, sublingual, intranasal, rectal or vaginal administration, inhalation, or transdermal application.

The composition is in a form appropriate to a planned route of administration, including sterile injectable solutions or powders, capsule, tablet, suspension, syrup, suppository, which are prepared by procedures well known in the art.

The invention relates also to the use of at least one peptide, lipopeptide, multi-epitopic peptide, expression vector as defined above, for the preparation of a vaccine for the prevention and/or the treatment of AIDS/HIV infection in an individual.

The invention provides also a peptide as defined above, as reagent for diagnosing the immune state of an individual with respect of the HIV virus.

According to the invention "diagnosing the immune state of an individual with respect to the HIV virus" refers to the detection, in said individual, of T CD4+ lymphocytes specific for the Tat protein. This detection makes it possible to follow the evolution of the HIV specific T CD4+ response during an HIV infection, an anti-HIV antiviral therapy or an anti-HIV vaccination.

According to an advantageous embodiment of said peptide, it is labelled and/or in the form of soluble-HLA-DR/peptide complex, preferably multimeric complexes such as tetrameric complexes comprising two peptides and two soluble HLA-DR molecules, each peptide being bound to one HLA molecule.

Labelled peptides for example with a fluorogenic molecule, are prepared according to standard procedures, known in the art.

Soluble-HLA-DR/peptide complexes are prepared and used according to the procedures as described for example in Novak et al. (J. Clin. Invest., 1999, 104, 63-67), Kuroda et al. (J. Virol., 2000, 74, 8751-8756) and the International PCT Applications WO 2004/014936 and WO 2006/027468.

The invention relates also to a method for diagnosing *in vitro* the immune state of an individual with respect to the HIV virus, comprising:
a) contacting a biological sample from said individual with a peptide as defined above, and
b) detecting T CD4⁺ cells specific for the Tat protein, by any appropriate means.

According to the invention, said biological sample comprises T CD4+ lymphocytes. Preferably, it is a blood sample. More preferably said biological sample is a suspension of peripheral blood mononuclear cells (PBMC), eventually depleted of CD8+ lymphocytes, or enriched in CD4⁺ lymphocytes by *in vitro* culture in the presence of the peptides according to the invention, which is prepared according to standard procedures known in the art.

According to the invention, T CD4⁺ cells specific for the Tat protein may be detected directly or indirectly. Direct detection may be obtained by contacting the biological sample with labelled HLA-DR/peptide multimeric complexes and detecting the cells labelled with the complexes, for example by flow cytometry analysis. Indirect detection may be obtained by contacting the biological sample with the peptide and detecting the specific T CD4+ cells by lymphocyte proliferation assay or cytokine (IL-2, IL-4, IL-5, IL-10, IFN-γ) assay. Cytokine assay may use immunoenzymatic (ELISA, RIA, ELISPOT) or flow cytometry (intracellular cytokine assay) techniques, as described previously in International PCT application WO 2006/027468.

The invention relates also to a kit for diagnosing the immune state of an individual with respect to the HIV virus, comprising a peptide as defined above.

A subject of the present invention is also a method for sorting HIV-specific T lymphocytes, characterized in that it comprises at least:
- contacting a cell suspension with one labelled HLA-DR/peptide multimeric complex, as defined above, and
- sorting the cells labelled with said HLA-DR/peptide multimeric complexes.

The invention provides also a non-immunogenic variant of the Tat peptide as defined above, comprising the substitution of at least one of the anchor residues for HLA-DR with an amino acid selected from the group consisting of: R, N, D, Q, E, H, K, P, S, T, G or A. The anchor residue which is substituted may be the leucine in position 43 (L43) and/or the isoleucine in position 45 (I45) of Tat protein sequence. The disruption of the T CD4+ epitope which is present in Tat 41-55 is expected to limit the immunogenicity of fused proteins, for example PTD fused proteins which target the cytosol for non immunological purposes.

A subject of the present invention is also a peptide, eventually modified, a multi-epitopic peptide, a fusion protein, a polynucleotide, an expression cassette, a vector, as defined above.

The invention encompasses in particular:
- a lipopeptide, a labeled peptide derived from the Tat peptide of the invention (immunogenic peptide or non-immunogenic variant),
- a multimeric complex, for example a tetramer formed by the association of soluble HLA-DR molecules with the Tat peptide of the invention.
- a multi-epitopic peptide consisting essentially of the concatenation of at least two different epitopes including the T CD4+ epitope of the Tat peptide of the invention,
- fusion proteins/fusion peptides comprising a peptide or a multi-epitopic peptide as defined above. The fusion moiety may be any protein of interest, for example an antigen including a Tat antigen such as for example a Tat toxoïd. The presence of the immunogenic Tat peptide in the fusion increases the immunogenicity of the antigen. In the opposite, the presence of the non-immunogenic variant of the Tat peptide limits the immunogenicity of fused proteins, for example PTD fused proteins, which target the cytosol for non immunological purposes. The additional presence of the Tat protein transduction domain in the fusion allows intracellular addressing of the protein. The fusion moiety may be separated from the multi-epitopic peptide backbone by a protease sensitive site, to facilitate the preparation of the peptide/multi-epitopic peptide. Fusion moiety which are useful for peptides preparation/detection are well known in the art and include for example glutathione-S-transferase (GST) and tags (hexa-histidine tag, B cell epitope tag),
- expression cassettes comprising at least a polynucleotide as defined above, under control of appropriate transcription and translation regulatory elements (promoter, enhancer, intron, initiation codon, stop codon, polyadenylation signal), and
- recombinant vectors comprising a polynucleotide according to the invention. Preferably, said vectors are expression vectors comprising an expression cassette as defined above, and
- prokaryotic or eukaryotic host cells modified by said polynucleotide or vector.

As used herein, a cell refers to a prokaryotic cell, such as a bacterial cell, or eukaryotic cell, such as a human, animal, plant or yeast cell.

The polynucleotides, recombinant vectors and modified cells as defined above are useful, for example for producing the peptides, multi-epitopic peptides and fusion proteins according to the present invention.

The practice of the present invention will employ, unless otherwise indicated, conventional methods of microbiology, molecular biology and immuno-biology within the skill of the art. Such techniques are explained fully in the litterature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986).

The polynucleotide sequence encoding the peptide, the multi-epitopic peptide or the fusion protein of the invention may be amplified from a cDNA template, by polymerase chain reaction with specific primers. Alternatively, it may be produced by chemical synthesis. Preferably, the codons of the coding sequences are chosen to favour the expression of said protein in the desired expression system. The recombinant vectors comprising said polynucleotide may be constructed and introduced in a host cell by the well-known recombinant DNA and genetic engineering techniques. The peptide or the multi-epitopic peptide of the invention, eventually included in a fusion protein, may be obtained by culturing the host cell (eukaryotic or prokaryotic) containing an expression vector comprising a polynucleotide sequence as defined above, under conditions suitable for the expression of said peptide, multi-epitopic peptide or fusion protein, and recovering said peptide, multi-epitopic peptide or fusion protein from the host cell culture. Advantageously, the presence of a tag allows affinity purification of the corresponding peptide, multi-epitopic peptide or fusion protein and the presence of a protease sensitive allows the isolation of the peptide or multi-epitopic peptide from the fusion protein.

Alternatively, the peptide or the multi-epitopic peptide of the invention may be prepared by chemical synthesis (Fmoc chemistry).

The lipopeptides may be prepared as described for example in the International PCT Application WO 99/40113 and WO 99/51630.

In addition to the preceding features, the invention further comprises other features which will emerge from the description which follows, which refers to exemplary embodiments of the peptide of the present invention, as well as to the accompanying drawings in which:

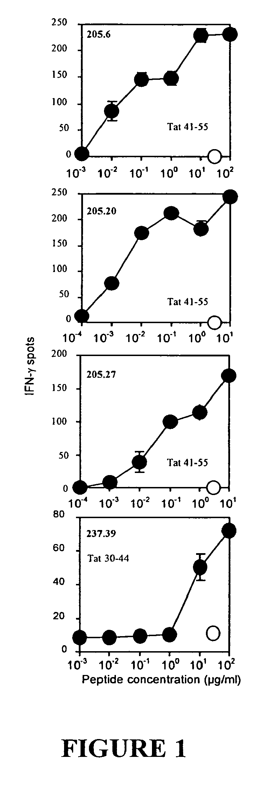

- figure 1 illustrates peptide-dose response of T cell lines induced by the selected Tat peptides. Peptide-specific CD4+ T cell lines were obtained after weekly stimulations by autologous mature dendritic cells loaded with the Tat peptide cocktail (10 µg/mL of each peptide). T cell lines (approximately 5 x 10³/well) were submitted to IFN-γ EliSpot assays using autologous PBMC (5 x 10⁴ to 10⁵/well) as Antigen Presenting Cells (APC). In the EliSpot assay, the peptides were used at 10 µg/mL. Experiments were performed in duplicate. Each panel shows the result for one T cell line

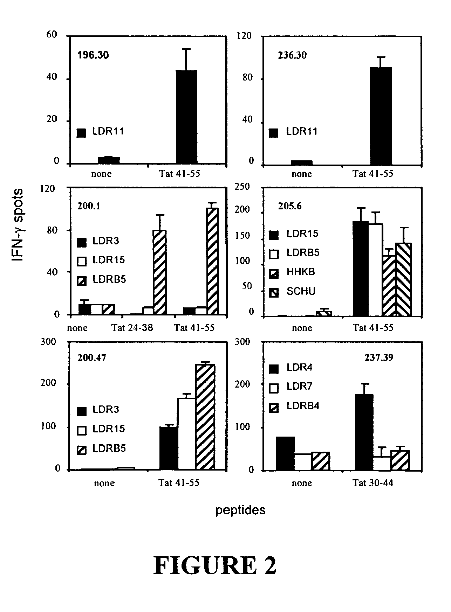

- figure 2 illustrates the restriction of the Tat Specific T cell lines. Peptide-specific CD4+ T cell lines were incubated with their appropriate peptide and L cells (3 x 10⁴ cells/well) transfected by one HLA-DR molecules (LDR3, LDR4, LDR7, LDR11, LDR15, LDRB4 or LDRB5) and submitted to IFN-γ EliSpot assays. Homozygote EBV cell lines SCHU (DR1501, DRB5) and HHKB (DR1301, DRB3) were also used as APCs for two T cell lines. Each value represents the mean spot number of duplicates.

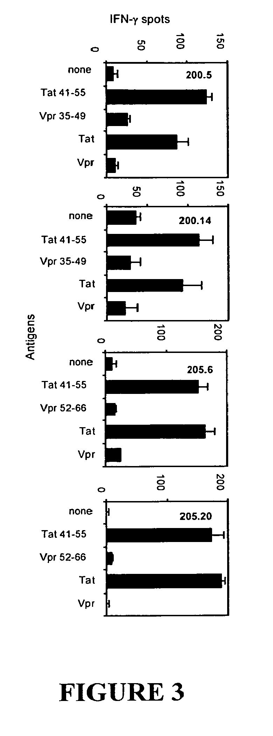

- figure 3 illustrates the presentation of the native Tat protein to the peptide-specific T cell lines. Peptide specific T cell lines (approximately 5 x 10³ cells/well) were incubated with immature dendritic cells (5 x 10³ per well) loaded with either the Vpr or Tat protein (3 µM) or the synthetic peptides (10µg/ml). Their specificity was investigated by IFN-γ EliSpot assays. Experiments were performed in duplicate.

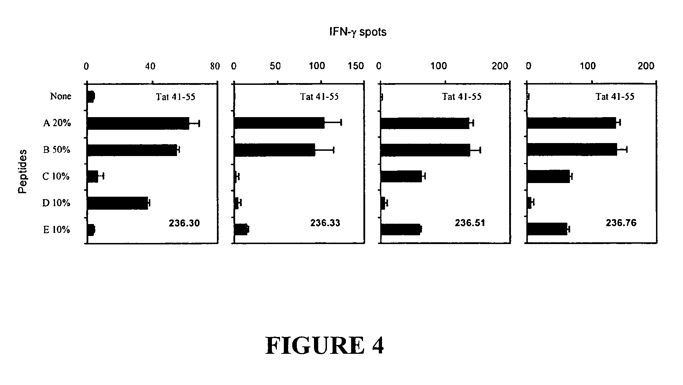

- figure 4 illustrates the recognition of Tat variants by peptide-specific T cell lines. T cell lines specific for the Tat peptide 41-55 were submitted to IFN-γ EliSpot assay. IFN-γ EliSpot assay was performed using autologous PBMC (5 10⁴ to 10⁵ cells/well) and peptide variants (10 µg/ml). Each bar represents the mean spot number of duplicates. Percentages indicated the frequency of the peptide sequence in clade B HIV-1 sequences (Los Alamos HIV databases http://hiv-web.lanl.gov)

### Example : Identification and characterization of Tat CD4+ T cell epitope

### 1) Material and Methods

### a) Peptides and proteins.

Overlapping purified Tat peptides derived from isolate LAI/IIIB (SEQ ID NO: 1) were provided by NeoMPS. The 86-residue-long Tat and the Vpr proteins (both from isolate LAI/IIIB) were synthesized using Fmoc-chemistry. After purification by reverse-phase HPLC, Tat and Vpr proteins were lyophilized and stored at - 20°C to prevent oxidation.

### b) HLA-DR peptide-binding assays

HLA-DR molecules were purified from homologous EBV cell lines by affinity chromatography using the monomorphic mAb L243 (Texier et al., J. Immunol., 2000, 164:3177-3184 ; Texier et al., Eur J Immunol., 2001, 31:1837-1846). The binding to HLA-DR molecules was assessed by competitive ELISA as previously reported (Texier et al., J. Immunol., 2000, 164:3177-3184 ; Texier et al., Eur J Immunol., 2001, 31:1837-1846). Unlabeled forms of the biotinylated peptides were used as reference peptides to assess the validity of each experiment. Their sequences and IC₅₀ values were the following: HA 306-318 (PKYVKQNTLKLAT; SEQ ID NO:8 ) for DRB1*0101 (2 nM), DRB1*0401 (31 nM), DRB1*1101 (14 nM) and DRB5*0101 (8 nM), YKL (AAYAAAKAAALAA; SEQ ID NO:9) for DRB1*0701 (7 nM), A3 152-166 (EAEQLRAYLDGTGVE; SEQ ID NO:10) for DRB1*1501 (270 nM), MT 2-16 (AKTIAYDEEARRGLE; SEQ ID NO:11) for DRB1*0301 (330 nM), B1 21-36 (TERVRLVTRHIYNREE; SEQ ID NO:12) for DRB1*1301 (203 nM), LOL 191-210 (ESWGAVWRIDTPDKLTGPFT ; SEQ ID NO:13) for DRB3*0101 (9 nM) and E2/E168 (AGDLLAIETDKATI; SEQ ID NO:14) for DRB4*0101 (20 nM). Peptide concentration that prevented binding of 50 % of the labelled peptide (IC₅₀) was evaluated. Data were expressed as relative affinity: ratio of the IC₅₀ of the peptide by the IC₅₀ of the reference peptide, which is a high binder to the HLA-DR molecule.

### c) Blood samples and HLA-DR genotyping

Blood cells were collected at the Etablissement Français du Sang (EFS) as buffy-coat preparations from anonym healthy donors after informed consent and following the guidelines of EFS. Peripheral blood mononuclear cells (PBMC) were isolated by density centrifugation on Ficoll-Hyperpaque gradients (SIGMA ALDRICH). HLA-DR genotyping was performed by using the Olerup SSP DRB1 typing kit (Olerup SSP AB).

### d) Generation and specificity of antigen-specific T cell lines

Immature and mature Dentritic cells (DC) were generated from plastic-adherent PBMC by a five-day culture in AIM-V medium supplemented with 1000 units/mL of rh-GmCSF (R&D SYSTEMS) and 1000 units/mL of rh-IL-4 (R&D SYSTEMS). LPS (SIGMA) (1 µg/mL) was used as maturation agent. CD4+ T lymphocytes were isolated by positive selection using an anti-CD4 monoclonal antibody through a MACS, as recommended by the manufacturer (MILTENYI BIOTECH). Their purity was assessed by cytometry. They were diluted in IMDM medium (INVITROGEN) supplemented with 24 mM glutamine, 55 mM asparagine, 150 mM arginine (all amino acids from SIGMA), 50 U/mL penicillin and 50 µg/mL streptomycin (INVITROGEN), and 10 % human serum (hereafter referred to as complete IMDM medium). Mature DC (5 x 10⁵) were incubated at 37 °C, 5 % CO₂, for 4 h in 1 mL of complete IMDM medium containing a mixture of peptides, each peptide being at a concentration of 10 µg/mL. Pulsed cells were washed and added at 10⁴ per round-bottom microwell to 10⁵ autologous CD4+ lymphocytes in 200 µL complete IMDM with 1000 U/mL of IL-6 (R&D systems, Abingdon, UK) and 10 ng/mL IL-12 (R&D Systems). 48 to 60 wells by donors were seeded by the co-culture of DC and CD4+ T lymphocytes. The CD4+ T lymphocytes were restimulated on days 7, 14 and 21 with autologous DC freshly loaded with the Tat peptide mixture, and were grown in complete IMDM medium supplemented with 10 U/mL IL-2 (R&D SYSTEMS) and 5 ng/mL IL-7 (R&D SYSTEMS). The stimulated CD4+ T cells were investigated for their peptide specificity by IFN-γ enzyme-linked immunospot (EliSpot) assays between days 28 to 32 as described previously (Cohen et al., J. Immunol., 2006, 176:5401-5408).

### 2) Results

### a) Multiple Tat peptides bound to multiple HLA-DR molecules

To identify the CD4+ T cell epitopes of HIV Tat, overlapping peptides encompassing their sequences were submitted to HLA-DR specific competitive ELISA binding assays. Because of the requirement of an aliphatic or aromatic residue in the N terminal part of the peptides to ensure their binding to HLA-DR molecules (Rammensee et al., Immunogenetics, 1995, 41:178-228; Sturniolo et al., Nat. Biotechnol., 1999, 17:555-561), overlapping peptides with these residues in position 1 to 5 were selected to optimize the screening. As a result, the Tat sequence was covered by 9 peptides (Table II). They overlapped by up to twelve residues and only excluded the 60-64 and 80-86 Tat sequences. Binding assays included the preponderant HLA-DR molecules previously used successfully to identify CD4+ T cell epitopes in multiple antigens (Texier et al., J. Immunol., 2000, 164:3177-3184 ; Zarour et al., Cancer Res., 2002, 62:213-218; Janjic et al., J. Immunol., 2006, 177:2717-2727 ; Castelli et al., Eur. J Immunol., 2007, 37:1513-1523). The data were presented as relative affinities (ratio of the IC₅₀ of the peptide by the IC₅₀ of the reference peptide, which is a high binder to the HLA-DR molecule) to easily compare their binding properties to high binder peptides that were used as reference.

**Table II: HLA-DR binding activity of overlapping HIV Tat peptides***

| **Peptides** | **Relative binding activity** | | | | | | | | | | **Number** |
|---|---|---|---|---|---|---|---|---|---|---|---|
| | **DR1** | **DR3** | **DR4** | **DR7** | **DR11** | **DR13** | **DR15** | **DRB3** | **DRB4** | **DRB5** | **HLA-DR** |
| **Tat 1-15** | 46057 | >302 | >3247 | >14697 | >6899 | **20** | 1656 | >714 | 2912 | >11242 | 1 |
| **Tat 6-20** | 27450 | >302 | >3247 | >14697 | 184 | **7** | 288 | >714 | >4932 | 699 | 1 |
| **Tat T 9-23** | 90909 | >302 | >3247 | >14697 | >6899 | >492 | >3731 | >714 | >4932 | 1854 | 0 |
| **Tat 24-38** | >66135 | **0.5** | >3247 | **96** | >6899 | **3** | **87** | **65** | **53** | >11242 | 6 |
| **Tat 30-44** | 195 | >302 | >3247 | **58** | **15** | 200 | >3731 | >714 | >4932 | 23 | 3 |
| **Tat 34-48** | 471 | **2** | >3247 | 40 | 8 | >492 | >3731 | >714 | 159 | 170 | 3 |
| **Tat 41-55** | 54545 | **48** | >3247 | >14697 | **83** | **0.5** | >3731 | >714 | >4932 | **0.3** | **4** |
| **Tat 45-59** | 12564 | >302 | >3247 | 6085 | 894 | **1** | >3731 | >714 | >4932 | 3429 | 1 |
| **Tat 65-79** | 72727 | >302 | >3247 | >14697 | 2280 | >492 | >3731 | >714 | >4932 | >11242 | 0 |

| | | | | | | | | | | | |
|---|---|---|---|---|---|---|---|---|---|---|---|
| * Relative activities inferior to 100 are in bold and correspond to active peptides. Means were calculated from at least three independent experiments | | | | | | | | | | | |

Most of the binding activities were found for peptides of the central part of the protein, namely Tat 24-38, 30-44, 34-48 and 41-55. The peptides bound to 3 to 6 HLA-DR molecules. These four peptides which bound at least to three HLA-DR molecules were then investigated for their capacity to prime *in vitro* a CD4+ T cell response.

### b) The T cell response specific for the Tat protein is mainly focused on one peptide only

Immunogenicity of the selected peptides was investigated by primary stimulation assays as previously described (Castelli et al., Eur. J. Immunol., 2007, 37:1513-1523). Peptides were tested in pools. CD4+ T lymphocytes were collected from 9 HIV seronegative donors, which exhibited a diversity of HLA-DR molecules, including all the HLA-DR molecules used in the binding experiments (Table III). Purified CD4+ T lymphocytes were seeded in 48 to 60 wells per donor and by peptide pool and stimulated by autologous peptide-loaded mature dendritic cells and a cocktail of cytokines. Rare peptide specific T cell precursors were amplified by three weekly stimulations and characterized by IFN-γ Elispot assays. IFN-γ Elispot assays was performed by using autologous PBMC as APC and peptides at 10 g/ml.

**Table III: CD4+ T cell response induced in vitro by Tat peptides**

| **HLA-DR Typing** | | | **Number of specific T cell lines** | | |
|---|---|---|---|---|---|
| **Donors** | **DRB1** | **2^{nd} DR** | **Tat 24-38** | **Tat 30-44** | **Tat 41-55** |
| P185 | 0701, 0404 | DRB4 | | | 2 |
| P191 | 0101, 1302 | DRB3 | | | 3 |
| P196 | 1101, 1104 | DRB3 | | | 1 |
| P200 | 0301,1501 | DRB3, DRB5 | 6 | | 7 |
| P205 | 1301,1501 | DRB3, DRB5 | | | 17 |
| P228 | 0701, 0901 | DRB4 | | | 1 |
| P237 | 0401, 0701 | DRB4 | 2 | 4 | |
| P236 | 1101,1301 | DRB3 | | | 5 |
| P249 | 1501, 1301 | DRB3, DRB5 | | | 47 |
| Responder frequency | | | 2/9 (22%) | 1/9 (11%) | 8/9 (89%) |

89 peptide specific T cell lines were identified. Because of the initial distribution of the CD4+ lymphocytes, most of the T cell lines are specific for one peptide only as illustrated by T cell lines #205.6, #205.20, #205.27 and #237.39 (Figure 1). This strongly suggests they derived from one CD4+ T cell precursor only. Interestingly, although the donors greatly differed in their HLA-DR molecules, almost all the T cell lines primed with the Tat peptide pool were specific for the Tat 41-55 peptide. Half-maximal stimulation of the T cell lines required a peptide concentration ranging from approximately 10⁻² to 10⁻¹ µg/ml (Figure 1). In agreement with the binding data, it was demonstrated using L cells transfected by an unique HLA-DR molecules, that the Tat 41-55 specific T cell response was restricted to DR11, DRB5 and DR3 alleles. Based on the data obtained using EBV cell lines with T cell line 205.6, it was probably also restricted to DR13. Restriction by the DR15 molecules, which was observed for few T cell lines was more suspicious as any binding to the immunopurified molecules was not detected. The corresponding T cell lines have been studied at high concentration of the peptide (10 µg/ml) only and could result from the T cell priming by the DRB5/Tat 41-55 complex. Therefore, the promiscuous Tat 41-55 peptide could be considered as public epitope as it is stimulating for a majority of individuals (Cohen et al., J. Immunol., 2006, 176:5401-5408; Castelli et al., Eur. J. Immunol., 2007, 37:1513-1523; Wang et al., Cancer. Immunol. Immunother., 2007, 56:807-818). Based on these data, it is expected that HLA-DR11, DR3, DR13 and DR15/DRB5 individuals are responders to Tat immunization and represents approximately 57 % of the Caucasian population.

Only few T cell lines were specific for other peptides (Tat 24-38 and 30-44) (Table III) and appeared less efficient to recognize them (Figure 1, T cell line #237.39). The fine specificity of the few T cell lines specific for Tat 24-38 was not investigated. Thus, both Tat 24-38 and Tat 30-44 appeared weakly immunogenic on the basis of the number of T cell lines induced and the frequency of responders, although they bound to multiple HLA-DR molecules. This is reminiscent to previous reports on HCV peptides (Castelli et al., Eur. J. Immunol., 2007, 37:1513-1523 and HLA-DP4 restricted peptides (Cohen et al., J. Immunol., 2006, 176:5401-5408; Wang et al., Cancer Immunol. Immunother., 2007, 56:807-818), showing that promiscuous or HLA-DP4 restricted peptides could be weakly immunogenic and suggested that it was due to inter-individual variation of the naive T cell repertoire.

Three peptides in the Tat protein were therefore immunogenic, the Tat 41-55 being T cell stimulating in almost all the donors.

### c) The T cell response to Tat peptides is restricted to multiple HLA-DR molecules

The HLA-DR molecules involved in the Tat peptide presentation were characterized by using L cells transfected with HLA-DR molecules corresponding to the typing of the donor, as APC. 29T cell lines were included in this study. Representative data were presented in Figure 2. CD4+ T cell lines specific for the Tat 41-55 were at least restricted to DR11 and DRB5. Some T cells are stimulated in a promiscuous manner and could also recognized the Tat 41-55 presented by DR3 and DR15. As illustrated by the T cell line #205.6, stimulation can also occurred with the DR13+ EBV cell line HHKB. Presentation to T cells of the Tat peptides therefore involved multiple HLA-DR molecules.

It is of interest to note that multiple T cell lines were restricted to HLA-DRB5. HLA-DRB5 is a 2^{nd} HLA-DR molecule encoded by a gene different from the main DRB1 gene. Its binding motif is very close to HLA-DRB1 molecule possessing a Gly at position β86 (Texier et al., Eur. J. Immunol., 2001, 31:1837-1846 ; Vogt et al., J. Immunol., 1994, 153:1665-1673). The 2^{nd} DR molecule seems to be expressed at a lower level at the surface of the APC as compared to HLA-DRB1 products (Cotner, T et al., J. Biol. Chem., 1989, 264:11107-11111; Stunz et al., J. Immunol., 1989, 143:3081-3086) but as shown in this work could elicit CD4+ T lymphocytes of various specificity as also described by others (Bach et al., J. Autoimmun., 1997, 10:375-386; Sotiriadou et al., , R Br.J. Cancer, 2001, 85:1527-1534; Sun et al., Cancer Immunol. Immunother., 2003, 52:761-770).

### d) Peptide-specific T cell lines recognized DC loaded with Tat protein

Tat protein and the control protein (Vpr) were chemically synthesized and not produced by recombinant technology. This limits the risk of LPS contamination. As shown in Figure 3, T cell lines specific for the Tat 41-55 which derived from two different donors (200 and 205) were stimulated by immature Dentritic cells (DC) loaded with the Tat protein but not by DC loaded by Vpr. By contrast, presentation of the Tat protein to the Tat 30-44 specific T cell line #237.39 could not be demonstrated. However, this T cell line appeared barely efficient to recognize the peptide (Figure 1).

Therefore, it was at least demonstrated that Tat 41-55 peptide-specific T cell lines recognized the native antigen processed by the DC. In contrast, the Tat 30-44 did not seem to correspond to a naturally processed peptide, although it was immunogenic for two healthy donors.

### e) Peptide-specific T cell lines can recognize other variants besides the LAI/IIIB sequence.

Sequences of the Tat peptides used in the binding assays and T cell priming experiments were retrieved from the LAI/IIIB isolate, corresponding to variant A of clade B. Because of the wide sequence variation of HIV, it was investigated whether the most frequent variants of the clade B (Table IV) were antigenic for the peptide-specific T cell lines.

**Table IV:clade B variant sequences of Tat 41-55 peptide**

| **Variant** | **Sequence** | **SEQ ID NO:** |
|---|---|---|
| **A** | KALGISYGRKKRRQR | 4 |
| **B** | KGLGISYGRKKRRQR | 3 |
| **C** | KGLGISYGSKKRRQR | 15 |
| **D** | KALGISNGRKKRRQR | 16 |
| **E** | KGLGIYYGRKKRRQR | 17 |

As shown in Figure 4, LAI/IIIB isolate (variant A) has a frequency of 20 % and all the T cells primed by this peptide recognized the most frequent variant (B) and to a lesser extent the three other variants (C, D and E).

## Claims

1. An isolated, immunogenic Tat peptide as anti-HIV vaccine, wherein said peptide consists of 11 to 15 consecutive amino acids of the peptidic fragment from positions 41 to 55 of Tat protein comprising at least positions 44 to 50, and wherein said Tat peptide consists essentially of a T CD4+ cell epitope and has a binding activity for at least three of the most prevalent HLA-DR molecules in the Caucasian population.

2. The peptide according to claim 1, wherein the amino acid sequence from positions 44 to 50 is GISYGRK (SEQ ID NO: 2).

3. The peptide according to claim 1 or claim 2, which is selected from the group consisting of: Tat 41-51, Tat 41-52, Tat 41-53, Tat 41-54, Tat 41-55, Tat 43-53, Tat 43-54 and Tat 43-55.

4. The peptide according to anyone of claims 1 to 3, which is from HIV-1 group M subtype B consensus sequence.

5. The peptide according to claim 4, which is of the sequence SEQ ID NO: 3.

6. The peptide according to anyone of claims 1 to 3, which is from HIV-1 group M subtype B variant A sequence.

7. The peptide according to claim 6, which is of the sequence SEQ ID NO: 4.

8. The peptide according to anyone of claims 1 to 7, which binds to at least three HLA-DR molecules selected from the group consisting of: HLA-DR1, HLA-DR3, HLA-DR4, HLA-DR7, HLA-DR11, HLA-DR13, HLA-DR15, HLA-DRB3, HLA-DRB4 and HLA-DRB5.

9. The peptide according to claim 8, which binds to HLA-DR3, HLA-DR11, HLA-DR13 and HLA-DRB5.

10. A vaccine composition for the prevention or the treatment of HIV infection/AIDS in an individual, comprising at least one immunogenic Tat peptide as defined in anyone of claims 1 to 9 and/or one polynucleotide encoding said peptide, and one pharmaceutically acceptable vehicle.

11. The vaccine composition according to claim 10, which comprises at least one immunogenic Tat peptide and at least one other HIV peptide including a T CD8+ and/or a B epitope, in the form of a peptide mixture, a multi-epitopic peptide or an expression vector encoding said peptides/multi-epitopic peptide.

12. The vaccine composition according to claim 11, which comprises a multi-epitopic peptide including the sequence of the Tat T CD4+ epitope as defined in anyone of claims 1 to 8 and at least one overlapping T CD8+ epitope selected from Tat 30-37, Tat 38-47 or Tat 36-50 and Tat 39-55 and/or the overlapping B epitope Tat 44-61.

13. The vaccine composition according to claim 11 or claim 12, wherein said multi-epitopic peptide further includes the Tat protein transduction domain having the sequence of Tat 49-57.

14. The vaccine composition according to anyone of claims 11 to 13, wherein said multi-epitopic peptide consists of Tat 30-55 or Tat 30-61.

15. The vaccine composition according to anyone of claims 10 to 14, which further comprises at least one HIV Nef, Env, RT, Vif or Vpr T CD4+ cell epitope, one universal T CD4+ epitope, and/or one B epitope consisting of a saccharide moiety.

16. A peptide as defined in anyone of claims 1 to 9, as reagent for diagnosing the immune state of an individual with respect of the HIV virus.

17. The peptide according to claim 16, which is labelled and/or in the form of soluble-HLA-DR/peptide complex.

18. A method for diagnosing *in vitro* the immune state of an individual with respect to the HIV virus, comprising:
a) contacting a biological sample from said individual with a peptide as defined in anyone of claims 1 to 9 and 17, and
b) detecting T CD4⁺ cells specific for the Tat protein, by any appropriate means.

19. A Kit for diagnosing the immune state of an individual with respect to the HIV virus, comprising a peptide as defined in anyone of claims 1 to 9 and 17.

20. The peptide as defined in anyone of claims 1 to 9 and 17.

21. A multi-epitopic peptide consisting essentially of the concatenation of the peptide as defined in anyone of claims 1 to 9 with at least another peptide as defined in anyone of claims 11 to 14.

22. A non-immunogenic variant of the peptide as defined in anyone of claims 1 to 9, comprising the substitution of at least one of the anchor residues for HLA-DR with an amino acid selected from the group consisting of: R, N, D, Q, E, H, K,P, S, T, G or A.

23. The non-immunogenic variant of claim 22, wherein said anchor residue is L43 or I45.

24. A fusion protein comprising a protein moiety fused to the peptide of anyone of claims 20, 22 and 23.

25. The fusion protein of claim 24 or the multi-epitopic peptide of claim 21, which further comprises the Tat protein transduction domain having the sequence of Tat 49-57.

26. A polynucleotide encoding the peptide of anyone of claims 20 to 23 or the protein of claim 24 or claim 25.

27. An expression vector comprising the polynucleotide according to claim 26.

28. A host cell which is modified by the polynucleotide according to claim 26 or the vector according to claim 27.
